# EUROPEAN PATENT APPLICATION

(11) **EP 1 201 764 A2**
(43) Date of publication of application: **02.05.2002**
(21) Application number: 01124850.7
(22) Date of filing: 18.10.2001
(51) Int. Cl.: C12Q 1/42

(54) **Assays for identifying phosphatase inhibitors**

(30) Priority: 31.10.2000 US 244539 P
(71) Applicant: Pfizer Products Inc., Groton, Connecticut 06340 (US)
(72) Inventor: Schaeffer, Eric, Groton, CT 06340 (US)
(74) Representative: Tesch, Rudolf, Dr.

(57) **Abstract**

The present invention relates to novel assays for the identification of agents that inhibit the catalytic activity of PTPbr7. The invention also provides pharmaceutical compositions comprising such agents identified using the assays of the invention. The invention further provides methods of treatment comprising administering such pharmaceutical compositions.

## Description

### FIELD OF THE INVENTION

The present invention relates to novel processes for determination of agents that inhibit the catalytic activity of protein tyrosine phosphatase PTPbr7. The invention also provides pharmaceutical compositions comprising agents identified using the assays of the invention. The invention further provides methods of treating neurodegenerative diseases by administering such pharmaceutical compositions.

### BACKGROUND OF THE INVENTION

Cellular signal transduction is a fundamental mechanism whereby extracellular stimuli are relayed to the interior of cells and subsequently these stimuli regulate diverse cellular processes. One of the key biochemical mechanisms of signal transduction involves the reversible phosphorylation of proteins.

Enzymes that mediate phosphorylation and dephosphorylation fall into two overall functional classes. The first class consists of protein kinases which transfer a phosphate moiety from adenosine triphosphate to protein substrates. The second class consists of protein phosphatases which hydrolyze phosphate moieties from phosphoryl protein substrates. These two converse functions of protein kinases and protein phosphatases balance and regulate the flow of signals in signal transduction processes.

Protein kinases and protein phosphatases are typically divided into three classes based on the amino acids they act upon. Some catalyze the addition or hydrolysis of phosphate on serine or threonine only, some catalyze the addition or hydrolysis of phosphate on tyrosine only and some catalyze the addition or hydrolysis of phosphate on serine, threonine and tyrosine.

Nerve growth factor (NGF) is a neurotrophic factor which mediates the survival, differentiation and maintenance of specific populations of neurons. For example, NGF has been shown to promote the survival of cholinergic neurons in the basal forebrain following a toxic insult. Cholinergic neurons have been shown to be particularly at risk for degeneration in Alzheimer's disease (AD). Thus, NGF treatment may have restorative effects in patients suffering from AD.

NGF itself is generally not thought of as an attractive therapeutic agent for treating neurodegenerative diseases of the central nervous system (CNS) because, like most proteins, NGF cannot traverse the blood brain barrier (BBB). Therefore, interest lies in molecules which mimic or facilitate NGF's activities yet are brain permeable.

The biological effects of NGF are mediated through a cell surface receptor with intrinsic tyrosine kinase activity. Following the binding of NGF to its receptor, a series of intracellular substrates are phosphorylated on tyrosine residues, which is necessary for the neurotrophic activity of this growth factor.

As would be appreciated by those skilled in the relevant art, and as mentioned above, growth factor signaling pathways generally involve checks and balances, e.g., a cascade of phosphorylations and dephosphorylations. Hence, as reported herein, one way to address finding molecules which mimic or facilitate NGF's activities is to identify a PTPase which acts as a negative regulator of NGF signaling and to screen for inhibitors of this enzyme. Inhibition of the PTPase would be expected to preclude the negative regulating of NGF by the PTPase and allow NGF to function; hence, the identified inhibitors would be expected to mimic or facilitate NGF's activities.

Accordingly, such inhibitors would be attractive therapeutic agents for treating any conditions or diseases in which proper NGF functioning plays a preventative role such as, for example, neurodegenerative diseases of the CNS, e.g., AD.

It has been reported by E. Sharma and P.J. Lombroso, "A Neuronal Protein Tyrosine Phosphatase Induced by Nerve Growth Factor," J. *Biol. Chem.* 270 (1): 49-53 (January 6, 1995) that the messenger ribonucleic acid (mRNA) for the protein tyrosine PTPase PTPbr7 is upregulated 8-fold in PC12 cells (a rat pheochromocytoma cell line derived from an adrenal tumor of neural crest origin) following treatment with NGF.

The cDNA encoding PTPbr7 has been cloned by three different groups, namely, by E. Sharma and P.J. Lombroso as referenced above (PC12-PTP1), by M. Ogata *et al.,* "cDNA Cloning and Characterization of a Novel Receptor-type Protein Tyrosine Phosphatase Expressed Predominately in the Brain," J. *Biol. Chem.* 270 (5): 2337-2343 (February 3, 1995) (PTPbr7) and by W. Hendricks et *al.,* "A novel receptor-type protein tyrosine phosphatase with a single catalytic domain is specifically expressed in mouse brain," *Biochem. J*. 305: 499-504 (1995) (PTP-SL). Ogata et al. and Hendricks *et al.* isolated the mouse clone as part of an effort to identify novel PTPases expressed in the brain while Sharma and Lombroso identified the rat clone while seeking to identify PTPases involved in NGF signaling. The Sharma and Lombroso original report contained a sequencing error, which was identified when these two other publications reported the cloning of the mouse enzyme. A human homolog has also been cloned (NC-PTPCOM1), and the sequence has been submitted (by P. Knyazev, Y. Cheburkin, Y. Knyazev and A. Ullrich, unpublished) to GenBank (Z79693).

The art lacks a simple, inexpensive and reliable basis for a high-throughput assay for finding agents which mimic or facilitate NGF's activities. The assays provided by the present invention utilize a PTPase, PTPbr7, which acts as a negative regulator of NGF signaling. Inhibitors of the PTPase would be expected to preclude the negative regulating of NGF by the PTPase and allow NGF to function, thus mimicking or facilitating NGF's activities. These agents would be useful, e.g., for the treatment of diseases or conditions related to signal transduction pathways employing PTPbr7, for the treatment of diseases or conditions related to the degeneration of cholinergic neurons, e.g., basal forebrain cholinergic neurons.

### SUMMARY OF THE INVENTION

The present invention provides methods for screening for or identifying agents that inhibit the dephosphorylation of a substrate by PTPbr7, pharmaceutical compositions comprising such agents, and methods of treating diseases related to impaired or suboptimal NGF signaling, or those related to the degeneration of cholinergic neurons, by administering such pharmaceutical compositions.

The methods of the invention comprise the steps of: interacting PTPbr7 with a substrate capable of being dephosphorylated by PTPbr7, in the presence and absence of a test agent (or putative inhibitor), in an assay medium comprising a reducing agent, for a period of time suitable for dephosphorylation to be substantially completed, detecting a change in the substrate or in the amount of "free" inorganic phosphate in the medium, and comparing the change in the substrate or in the amount of "free" inorganic phosphate in the medium, where an inhibitor is a test agent that resulted in decreased change in the substrate, or decreased "free" inorganic phosphate in the medium, depending upon the substrate selected, and the desired readout methodology. Preferably, the decrease in released inorganic phosphate in the presence of a test agent acting as an inhibitor is from about 10% to about 100%, preferably between from about 35% to about 100%, and especially preferably at least about 50%, compared with the inorganic phosphate released in the absence of the agent (inhibitor).

Accordingly, the present invention provides methods for identifying agents that inhibit the catalytic activity of PTPbr7, comprising the steps of:
(a) combining, in a first cocktail, an amount of a PTPbr7, an amount of a substrate capable of being dephosphorylated by PTPbr7, and an amount of a test agent, in an assay buffer comprising a reducing agent;
(b) preparing a second cocktail comprising all of the ingredients of said first cocktail except for said test agent;
(c) incubating said first and second cocktails under conditions suitable to allow for a substantial amount of dephosphorylation of said substrate by said PTPbr7;
(d) quantitating the amount of dephosphorylation in each of said cocktails; and
(e) comparing said amounts of dephosphorylation;
where a PTPbr7 inhibitor is a test agent whose presence results in less dephosphorylation than its absence.

The present invention also provides methods for identifying agents that inhibit the catalytic activity of PTPbr7, comprising the steps of:
(a) combining, in a first cocktail, an amount of a PTPbr7, an amount of a substrate capable of being dephosphorylated by PTPbr7, and an amount of a test agent, in an assay buffer comprising a reducing agent;
(b) preparing a second cocktail comprising all of the ingredients of said first cocktail except for said test agent;
(c) incubating said first and second cocktails under conditions suitable to allow for a substantial amount of dephosphorylation of said substrate by said PTPbr7;
(d) terminating said incubation;
(e) quantitating the amount of dephosphorylation in each of said cocktails; and
(f) comparing said amounts of dephosphorylation;
where a PTPbr7 inhibitor is a test agent whose presence results in less dephosphorylation than its absence.

In a preferred embodiment, full length PTPbr7 is used. The source of the full length PTPbr7 is preferably rat or mouse, and is particularly preferably human. In another preferred embodiment, a truncated PTPbr7 is used. In yet another preferred embodiment, the catalytic domain of PTPbr7 is used. In a further embodiment, a mutated form of PTPbr7 is used. In a particularly preferred embodiment, a full length, truncated, or mutated PTPbr7 fused to a heterologous sequence is used, where the heterologous sequence can be used for, e.g., affinity, purification and detection. In any given fusion protein, the PTPbr7 may be fused either N- or C-terminally to the fusion partner. In a particularly preferred embodiment, a glutathione-S-transferase (GST)-PTPbr7 fusion protein is used. In another particularly preferred embodiment, six-histidine residue tag (6Xhis)-PTPbr7 fusion protein is used.

In a preferred embodiment, the substrate capable of being dephosphorylated by PTPbr7 is a phosphoprotein. In a preferred embodiment, the substrate capable of being dephosphorylated by PTPbr7 is phospho-MAP kinase. In another preferred embodiment, the substrate capable of being dephosphorylated by PTPbr7 is a peptide containing a phosphotyrosine residue. In another preferred embodiment, the substrate capable of being dephosphorylated by PTPbr7 is a peptide containing a phosphotyrosine residue selected from [ENDpYINASL] (a nonapeptide derived from a highly conserved region of the T-cell phosphatase TC.PTP, Daum et *al*., *Analytical Biochemistry* 211: 50 (1993)) and [DHTGFLTEpYVATRW] (MAP kinase phosphopeptide). In another preferred embodiment, the substrate capable of being dephosphorylated by PTPbr7 is a small molecule mimetic, and preferably is para-nitrophenylphosphate or is O-methylfluorophosphate, and particularly preferably is O-methylfluorophosphate.

In a preferred embodiment, the test agent is a molecule capable of traversing the BBB.

In a preferred embodiment, the substrate is para-nitrophenylphosphate, and the phosphate groups released from the substrate into the medium are detected and quantitated using spectrophotometric methods.

In another preferred embodiment, the substrate is a peptide containing a phosphotyrosine residue, and the residual phosphotyrosine of the substrate is quantitated using an anti-phosphotyrosine antibody.

In another preferred embodiment, the substrate is a biotinylated form of a peptide having a phosphotyrosine residue and is bound to streptavidin-coated wells, PTPbr7 is GST-PTPbr7, and the amount of substrate is quantitated using an anti-phosphotyrosine antibody.

In yet another preferred embodiment, the substrate is O-methylfluorophosphate, the liberation of the phosphate groups by hydrolysis of the substrate causes an increase in the fluorescent emission of the resultant O-methylfluoroscene, which is detected using fluorometric analysis.

In a preferred embodiment, PTPbr7 is a GST-PTPbr7 fusion protein, the substrate is a peptide having a phosphotyrosine residue, the dephosphorylation is stopped by adding a solution comprising the dye malachite green, ammonium molybdate and Tween®-20, waiting for a suitable period of time, e.g., about 15min, and then quantitating the phosphate groups released into the medium using spectrophotometric methods.

The present invention also provides cell-based methods for identifying inhibitors of PTPbr7.

In a preferred embodiment, PC12 cells are transfected with PTPbr7, preferably rodent or human PTPbr7, and overexpressing cell lines are identified, e.g., by Northern Blot analysis, and these cell lines are then treated with a suitable amount of NGF (e.g., from about 10 ng/mL to about 50 ng/mL) for a suitable period of time, e.g., several days (such as 3-10 days), in the presence or absence of the test agent, and the degree of neurite outgrowth is quantitated.

In another preferred embodiment, the PC12 cell lines are prepared and treated with NGF substantially as described above, and MAP kinase phosphorylation (then subsequent dephosphorylation) is monitored as a readout of the inhibition of PTPbr7.

In yet another preferred embodiment, the PC12 cell lines are prepared substantially as described above, and genes whose expression are known to be induced by NGF can be measured as a readout of the inhibition of PTPbr7.

In yet another preferred embodiment, the PC12 cell lines are prepared substantially as described above, and the survival of relevant populations of neurons can be examined as a readout of the inhibition of PTPbr7 or the removal of the negative regulation by PTPbr7 of NGF signaling.

In yet another embodiment, primary neurons, preferably, basal forebrain neurons are cotransfected with PTPbr7 and lac Z, then treated with NGF and/or bovine-derived neurotrophic factor (BDNF) for a suitable period of time, e.g., several days, in the presence or absence of the test agent, and the degree of neurite outgrowth is quantitated.

In another embodiment, the present invention provides pharmaceutical compositions comprising an inhibitor identified using the methods of the invention, or a pharmaceutically acceptable salt of the inhibitor, and a pharmaceutically acceptable carrier, vehicle, or diluent. In a preferred pharmaceutical composition, the inhibitor is a molecule capable of traversing the BBB.

In yet another embodiment, the present invention provides methods of treating diseases or conditions related to the degeneration of cholinergic neurons by administering to a mammal in need of such treatment a pharmaceutical composition of the invention. In a particularly preferred method, the disease or condition is related to the degeneration of basal forebrain neurons. In another preferred method, the disease or condition is related to signal transduction pathways employing PTPbr7. In yet another preferred method, the disease or condition is a neurodegenerative disease or condition of the CNS. In yet another particularly preferred method, the disease or condition is related to impaired or suboptimal NGF signaling.

Those skilled in the art will fully understand the terms used in the description and attendant claims to describe the present invention; nonetheless, the following terms used herein are as described immediately below.

"Pharmaceutically acceptable" means that the carrier, diluent, vehicle excipients, and/or salt must be compatible with the other ingredients of the formulation, and not deleterious to the recipient thereof.

"Pharmaceutically acceptable salt(s)" includes salts of acidic or basic groups which may be present in the compounds suitable for use in the pharmaceutical compositions and methods of the present invention, e.g., the inhibitors of the catalytic activity of PTPbr7 identified by the assays of the present invention. For example, pharmaceutically acceptable salts include sodium, calcium and potassium salts of carboxylic acid groups and hydrochloride salts of amino groups. Other pharmaceutically acceptable salts of amino groups are hydrobromide, sulfate, hydrogen sulfate, phosphate, hydrogen phosphate, dihydrogen phosphate, acetate, succinate, citrate, tartrate, lactate, mandelate, methanesulfonate (mesylate) and p-toluenesulfonate (tosylate) salts. A preferred salt is the citrate.

"Pharmaceutical composition" refers to any composition comprising an agent(s) provided by the present invention formulated in any suitable fashion such as any suitable formulation well known in the art and a pharmaceutically acceptable carrier, vehicle, or diluent. Any suitable route of administration of the pharmaceutical composition may be used including, for example, injection, transmucosal, oral, inhalation, ocular, rectal, long acting implantation, liposomes, emulsion, and sustained release means.

"Treating," "treat" or "treatment" includes, *inter alia,* preventative (e.g., prophylactic), palliative and curative treatment, including, for example, the prevention, reduction and reversal of neurodegeneration.

BME is betamercaptoethanol; BSA is bovine serum albumin; °C is degrees Centigrade; % is percent; DMSO is dimethylsulfoxide; DTT is dithiothreitol; EGTA is ethylene glycol bi(β-aminoethyl ether) *N,N,N',N' -* tetraacetic acid; KCI is potassium chloride; MgCl₂ is magnesium chloride; mg/mL is milligram or milligrams per milliliter or milliliters; min is minute or minutes; mM is millimolar (concentration); nm is nanometer or nanometers; pmol is picomole or picomoles; RT-PCR is reverse transcriptase-polymerase chain reaction; µL is microliter; µM is micromolar (concentration).

American Peptide Company is located in Sunnyvale, California (USA).

Amersham® is located in Arlington Heights, Illinois (USA).

Life Technologies is located in Gaithersburg, Maryland (USA).

Packard Instrument Company is located in Meriden, Connecticut (USA).

PanVera Corporation is located in Madison, Wisconsin (USA).

SIGMA®-Aldrich is located in St. Louis, Missouri (USA).

Strategene is located in La Jolla, California (USA).

All of the documents, including collections of documents and protocols, cited herein are incorporated by reference herein in their entireties.

### DETAILED DESCRIPTION OF THE INVENTION

Because NGF mediates the survival, differentiation and maintenance of specific populations of neurons, e.g., has been shown to promote the survival of cholinergic neurons in the basal forebrain following a toxic insult, and cholinergic neurons have been shown to be particularly at risk for neurodegeneration, e.g., in AD, and NGF itself is generally not thought of as an attractive therapeutic agent for treating neurodegenerative diseases of the CNS because, like most proteins, NGF cannot traverse the BBB, there is a need in the art for molecules which mimic or facilitate NGF's activities yet are brain permeable.

As described herein, because PTPbr7, a tyrosine phosphatase, is transcriptionally upregulated in PC12 cells in response to NGF treatment (Sharma and Lombroso), and PTPbr7 is a negative regulator of NGF signaling (see EXAMPLE 1), diseases or conditions related to impairment of or suboptimal NGF signaling, and consequently the cascade of intracellular phosphorylations and dephosphorylations which is necessary for the neurotrophic activity of NGF, may be treated with agents which inhibit the catalytic activity of PTPbr7. Hence, as would be appreciated by those skilled in the art, simple, inexpensive, reliable, accurate assays that allow the screening for, or identification of, inhibitors of PTPbr7 and, in particular, the identification of small molecule inhibitors that can traverse the BBB, would enable the art to develop novel therapeutics that would be useful in treating any diseases or conditions in which proper NGF functioning plays a preventative role such as, for example, neurodegenerative diseases of the CNS, e.g., AD. Such assays would also form the basis, for instance, of high-throughput assays to identify PTPbr7 inhibitors.

The present assays are directed to the detection of either an increase or a decrease in the extent of substrate phosphorylation by PTPbr7 as indicative of either increased or decreased PTPbr7 catalytic activity.

Therefore, the present invention provides screening methods for identifying agents that inhibit the catalytic activity of PTPbr7, comprising the steps of:
(a) combining, in a first cocktail, an amount of a PTPbr7, an amount of a substrate capable of being dephosphorylated by PTPbr7, and an amount of a test agent, in an assay buffer comprising a reducing agent;
(b) preparing a second cocktail comprising all of the ingredients of said first cocktail except for said test agent;
(c) incubating the first and second cocktails under conditions suitable to allow for a substantial amount of dephosphorylation of said substrate by said PTPbr7;
(d) quantitating the amount of dephosphorylation in each of said cocktails; and
(e) comparing said amounts of dephosphorylation;
where a PTPbr7 inhibitor is a test agent whose presence results in less dephosphorylation than its absence.

Those skilled in the art will understand based upon the present description, including the EXAMPLES hereinbelow, and the several aspects and embodiments of the assays of the present invention described in the present description and in the attendant claims, how to select a suitable amount of enzyme, substrate, reagent, and the like, to achieve any particular desired result, as the case may be.

Any suitable PTPbr7 or active fragment or portion thereof, e.g., full length, truncated, or mutated, can be used in the screening methods of the present invention. In addition, the PTPbr7 may be derived from either human or non-human sources, with rodent sources being preferable, specifically, rat and mouse, and human sources being particularly preferable, e.g., from human cell lines. As with human cell lines sources, non-human PTPbr7 can be isolated from non-human cell lines, e.g., PC12 cells. Typcially, those skilled in the art would employ a recombinant PTPbr7, but use of any suitable endogenous PTPbr7 is also intended to be within the scope of the present invention.

Any suitable fusion protein comprising a PTPbr7 can be used in the methods of the present invention. The fusion proteins or enzymes used in the present methods comprise the catalytic domain of a PTPbr7 fused to a heterologous sequence. The heterologous sequence can be used for affinity purification and detection. Any suitable fusion partner may be used in the methods of the present invention, and the PTPbr7 can be fused either N-terminally or C-terminally to such fusion partner. A preferred fusion partner is a 6Xhis, which can be purified on an immobilized metal affinity column and detected with an anti-tetra-his antibody. A particularly preferred fusion partner is GST from *E*. *coli*, which can be purified on a glutathione affinity column and detected with antibodies to GST.

Any suitable substrate capable of being dephosphorylated by PTPbr7 can be used in the screening methods of the present invention. As those skilled in the art will appreciate, either the phosphate group(s) released from the substrate or the extent of phosphotyrosine remaining on the peptide containing the phosphotyrosine residue(s) can be measured as a readout of the extent of dephosphorylation by PTPbr7 in the absence and presence of the test agent, as the case may be, depending upon what type of substrate is employed. In addition, the substrate may be bound to the reaction vessel using conventional methods, e.g., the substrate is a biotinylated form of a peptide having a phosphotyrosine residue and the walls of the reaction vessel are coated with streptavidin, which would then enable the use of a detection reagent, e.g., an anti-phosphotyrosine antibody, to quantitate the number of phosphotyrosine residues remaining after the dephosphorylation reaction is terminated, e.g., by rinsing the enzyme and the reaction products out of the reaction vessel, e.g., using a suitable buffer such as the assay buffer selected for the reaction. Moreover, the antibodies selected for use by those skilled in the art, e.g., to quantitate the number of phosphotyrosine residues remaining after the dephosphorylation reaction is terminated, can be conjugated to any suitable enzyme such as, for example, horseradish peroxidase (HRP), alkaline phosphatase (AP), or the like, then interacted with a suitable substrate for the chosen enzyme, as is well known in the art.

Preferred substrates include small molecule mimetics which can pass hrough the BBB. A preferred small molecule mimetic is para-nitrophenylphosphate. The phosphate group released from para-nitrophenylphosphate can be quantitated using any suitable method such as, for example, spectrophotometric analysis.

Any suitable reagents and conditions can be used in the assays of the present invention, and those skilled in the art will understand based on the present description and examples provided herein, how to select such conditions, depending upon the specific reagents employed and desired result sought. Any suitable buffer known by those skilled in the art which permits dephosphorylation reactions can be used in the present assays. Suitable buffers would include those which comprise a buffer, e.g., 10 mM Tris or Hepes, a salt, e.g., 150 mM NaCI, and a detergent, e.g., 0.05% Tween-20. For example, in an embodiment where Malachite Green is used as the readout, the enzyme buffer comprises 50 mM Tris, 0.15M NaCI, 5 mM DTT, and 0.1 % BSA. In addition, as those skilled in the art will appreciate, the enzyme is stabilized (from degradation) by the inclusion of a suitable reducing, e.g., DTT or BME. Preferably, the reducing agent is present at a final concentration of from 1 mM to about 50 mM, with 5 mM being especially preferred.

Those skilled in the art will also understand how to optimize any given assay in terms of the pH of the dephosphorylation reaction; however, as a general guide which should be suitable for most, if not all, reactions, depending, once again on the choice of reagents, the amounts of those reagents, and the desired result, a suitable pH range would be from about pH 5.0 to about pH 8.0, with a pH of about 7.4 being generally most preferred. Those skilled in the art would also know from conventional methods which reagents to use to adjust the pH in any given direction to avoid any unwanted interference with the dephosphorylation reaction.

Any suitable period of time can be selected for the dephosphorylation reaction such as, for example, a period of time from about 5 min to about 90 min., e.g., 30 min. Those skilled in the art will understand from conventional methods how to determine or "titrate" the period of time allowed for dephosphorylation against the desired extent of dephosphorylation for any particular individual assay, to facilitate a rapid yet reliable and accurate identification of inhibitors of PTPbr7.

Those skilled in the art will also understand how to optimize any given assay in terms of the temperature at which the dephosphorylation reaction is run; however, as a general guide which should be suitable for most, if not all, reactions, depending, once again on the choice of reagents and the desired result, a suitable temperature range would be from about room temperature, about 25°C, to about 37°C, with a temperature closer to room temperature being generally most preferred. Those skilled in the art would also know what conventional methods to use to adjust the temperature to within the desired range either before or during the selected period of time for dephosphorylation.

Any suitable reaction vessel can be used in the methods of the present invention. The reaction vessel may be of any suitable design, e.g., shape, surface area, volume, and the like, and comprised of any suitable material. Suitable reaction vessels include, for example, microtiter plates, e.g., 48-well or 96-well microtiter plates, e.g., COSTAR #3690 plate. In addition, the subject assays can be performed on a desired larger scale, for example, by using an automated, e.g., robotic, system.

Aspects of the present invention are now provided as examples of choices of reagents that one skilled in the art may decide to employ in the assays of the invention.

In a first aspect, the method for identifying an agent that inhibits the catalytic activity of PTPbr7, comprises the steps of:
(a) combining, in a first cocktail, an amount of a PTPbr7, an amount of a substrate capable of being dephosphorylated by PTPbr7, and an amount of a test agent, in an assay buffer comprising a reducing agent;
(b) preparing a second cocktail comprising all of the ingredients of said first cocktail except for said test agent;
(c) incubating said first and second cocktails under conditions suitable to allow for a substantial amount of dephosphorylation of said substrate by said PTPbr7;
(d) terminating said incubation;
(e) quantitating the amount of dephosphorylation in each of said cocktails; and
(f) comparing said amounts of dephosphorylation;
where a PTPbr7 inhibitor is a test agent whose presence results in less dephosphorylation than its absence.

Preferred individual embodiments within the first aspect include those wherein:
(1) PTPbr7 is full length;
(2) PTPbr7 is recombinant rodent full length;
(3) PTPbr7 is recombinant mouse or rat full length;
(4) PTPbr7 is a fusion protein comprising the catalytic domain of PTPbr7 and a heterologous sequence;
(5) PTPbr7 is a fusion protein comprising the catalytic domain of PTPbr7 and a heterologous sequence, where PTPbr7 is fused N-terminally to the heterologous sequence;
(6) PTPbr7 is a fusion protein comprising the catalytic domain of PTPbr7 and a heterologous sequence, where PTPbr7 is fused C-terminally to the heterologous sequence;
(7) PTPbr7 is a fusion protein comprising the catalytic domain of PTPbr7 and a heterologous sequence, and the heterologous sequence is glutathione-S-transferase or a six-histidine residue tag;
(8) the substrate is a small molecule mimetic;
(9) the substrate is a small molecule mimetic, i.e., para-nitrophenylphosphate;
(10) the terminating is by adding a base to the assay buffer;
(11) the terminating is by adding NaOH to the assay buffer, where the final concentration of the NaOH is 0.95N;
(12) the substrate is a phosphoprotein, or a peptide having a phosphotyrosine residue;
(13) the substrate is a peptide having a phosphotyrosine residue selected from [ENDpYINASL] and [DHTGFLTEpYVATRW].
(14) the substrate is a phosphoprotein, i.e., MAPK-P.
(15) suitable conditions for dephosphorylation are a temperature of from about room temperature to about 37°C, and an incubation time of from about 5 min to about 90 min;
(16) the assay buffer comprises a buffer, a salt, and a detergent;
(17) the assay buffer comprises Tris or Hepes, NaCI, and Tween-20;
(18) the assay buffer comprises 50 mM Tris or 50 mM Hepes, 150 mM NaCI, and 0.05% Tween-20;
(20) the reducing agent is DTT or BME; and/or (as the case may be based upon the present description)
(21) the substrate is a phosphoprotein, or a peptide having a phosphotyrosine residue, the amount of assay buffer is about 25µL, the assay buffer comprises about 50 mM Tris, about 0.15 M NaCI, about 5 mM DTT, and about 0.1% BSA, at about pH 7.4, the terminating is by adding a stop solution comprising malachite green dye, ammonium molybdate, and Tween-20 to said cocktails, and waiting about 15 min, and the quantitating is by measuring the optical density at 620 nm of each of the stopped cocktails using a spectrophotometer, and comparing the optical density values with a set of standards comprising varied amounts of inorganic phosphate prepared in the stop solution.

In a second aspect, the method for identifying an agent that inhibits the catalytic activity of PTPbr7, comprises the steps of:
(a) combining, in a first cocktail, an amount of a PTPbr7, an amount of a substrate capable of being dephosphorylated by PTPbr7, and an amount of a test agent, in an assay buffer comprising a reducing agent;
(b) preparing a second cocktail comprising all of the ingredients of said first cocktail except for said test agent;
(c) incubating said first and second cocktails under conditions suitable to allow for a substantial amount of dephosphorylation of said substrate by said PTPbr7;
(d) quantitating the amount of dephosphorylation in each of said cocktails; and
(e) comparing said amounts of dephosphorylation;
where a PTPbr7 inhibitor is a test agent whose presence results in less dephosphorylation than its absence.

Preferred individual embodiments within the second aspect include those wherein:
(1) PTPbr7 is full length;
(2) PTPbr7 is recombinant rodent full length;
(3) PTPbr7 is recombinant mouse or rat full length;
(4) PTPbr7 is a fusion protein comprising the catalytic domain of PTPbr7 and a heterologous sequence;
(5) PTPbr7 is a fusion protein comprising the catalytic domain of PTPbr7 and a heterologous sequence, where PTPbr7 is fused N-terminally to the heterologous sequence;
(6) PTPbr7 is a fusion protein comprising the catalytic domain of PTPbr7 and a heterologous sequence, where PTPbr7 is fused C-terminally to the heterologous sequence;
(7) PTPbr7 is a fusion protein comprising the catalytic domain of PTPbr7 and a heterologous sequence, and the heterologous sequence is glutathione-S-transferase or a six-histidine residue tag;
(8) the substrate is a small molecule mimetic;
(9) the substrate is a small molecule mimetic, i.e., O-methylfluorophosphate;
(10) the substrate is a small molecule mimetic, i.e., O-methylfluorophosphate, and the quantitating is by measuring the excitation at 485 nm and fluorescent emission at 535 nm of each of said cocktails using a fluorometer; and/or (as the case may be, based upon the present description)
(11) suitable conditions for dephosphorylation are a temperature of from about room temperature to about 37°C, and an incubation time of from about 5 min to about 90 min.

In a third aspect, the PTPbr7 is a recombinant full length mouse PTPbr7 (see, Ogata et al. and W. Hendriks et al.; alternatively, for mRNA, complete cds, see GenBank Accession Number AF041866, S.N. Silbiger *et al*., unpublished), the substrate is a peptide having a phosphotyrosine residue, the assay buffer comprises 10 mM Tris, 150 mM NaCI, 5 mM DTT, and 0.05% Tween-20, the test agent is a compound, the dephosphorylation reaction is allowed to proceed for about 60 min at room temperature, whereupon it is terminated by the addition of a stop solution comprising malachite green, ammonium molybdate, and Tween-20 (see Harder *et al., Biochem. J*., 298: 395 (1995), the malachite green undergoes a color change in the presence of inorganic phosphate)), and the amount of phosphate released from the substrate by the action of PTPbr7, in the absence and presence of the test agent, is quantitated using spectrophotometric analysis at 620 nm, where less absorbance (optical density) at 620 nm in the presence of a test agent compared with its absence indicates that the test agent is inhibiting the catalytic activity of PTPbr7.

In a fourth aspect, the PTPbr7 is a recombinant full length mouse PTPbr7 (see, Ogata et al. and W. Hendriks et al.; alternatively, for mRNA, complete cds, see GenBank Accession Number AF041866, S.N. Silbiger *et al*., unpublished), the substrate is O-methyl fluorophosphate, the assay buffer comprises 50 mM Hepes (pH 7.2), 100 mM KCI, 2.5 mM EGTA, 2.5 mM MgCl₂, 1 mg/mL BSA, and 5 mM DTT, the dephosphorylation reaction is allowed to proceed for about 10 min at room temperature, and the increase in fluorescent emission of the resulting dephosphorylated O-methyl fluorescene is measured by excitation at 485 nm and emission at 535 nm using a Victor II plate reader (Packard), in the absence and presence of the test agent, where a test agent acting as an inhibitor of the catalytic activity of PTPbr7 will cause a decrease in the production of O-methyl fluorescene which is measured as a decrease in the fluorescent signal compared to the signal observed in the absence of the test agent.

In a fifth aspect of the present invention, PTPbr7 is a GST-PTPbr7 fusion protein comprising the catalytic domain of PTPbr7, and this is added to a streptavidin-coated well of a 96-well microtiter plate, where the well has prebound (through the streptavidin) to it a biotinylated form of a peptide having a phosphotyrosine residue (the substrate) and contains an amount of assay buffer comprising 10 mM Tris, 150 mM NaCI, and 0.05% Tween-20, and 5 mM DTT, in the presence and absence of a test compound, an incubation is allowed to occur at room temperature for about 15 min, the reaction is terminated by rinsing the PTPbr7 and any reaction products out of the well using an amount of the assay buffer, an amount of an anti-phosphotyrosine antibody conjugated to HRP is then added to the well and incubated for about 30 min, and bound antibody is detected with an HRP substrate using standard procedures, where the amount of bound antibody, in the presence and absence of the test compound, is a measure of how much of the substrate remains phosphorylated after exposure to the PTPbr7, and therefore, is a measure of to what extent the test compound inhibited the catalytic activity of the PTPbr7.

The screening methods of the present invention also include cell-based assays for the identification of PTPbr7 inhibitors.

In a first aspect, the methods of screening for agents that inhibit the catalytic activity of PTPbr7, comprise the steps of: exposing cells or a cell line expressing PTPbr7 and capable of responding to NGF, to an amount of NGF, in the presence and absence of a test agent, under conditions suitable for a detectable NGF response to occur in the presence of a PTPbr7 inhibitor, detecting said response, and comparing said response, where a PTPbr7 inhibitor is a test agent whose presence results in more of an NGF response than its absence.

Preferred individual embodiments within the first aspect include those wherein:
(1) the cell line is a PC12 cell line, or a PC12 cell line stably transfected with a PTPbr7, and said cells are primary neurons, or primary neurons cotransfected with a cDNA clone of a PTPbr7 and lac Z;
(2) the cells are primary neurons, i.e., cholinergic neurons;
(3) the cells are cholinergic neurons, i.e., basal forebrain neurons;
(4) the cell line is a transfected PC12 cell line that overexpresses PTPbr7, and the cells are primary neurons transfected to overexpress PTPbr7;
(5) the cells are primary neurons cotransfected with PTPbr7 and lacZ, and overexpress PTPbr7;
(6) the NGF response is neurite outgrowth;
(7) the NGF response is survival;
(8) the NGF response is the expression of any gene induced by NGF; and/or (as the case may be, based upon the present description)
(9) the NGF response is MAPK phosphorylation.

As mentioned above, and more specifically, for example, a cDNA clone of mouse full length PTPbr7 is introduced into PC12 cells (which have endogenous PTPbr7), e.g., by transfection, stable cell lines are selected using conventional techniques based on the transfected vector (e.g., G418 selection), and the overexpression of PTPbr7 in these stable lines is verified by Northern Blot analysis. [Those skilled in the art will understand from standard texts in the art such as, for example, Current Protocols in Molecular Biology (CPMB, Eds. F. Ausubel *et al*., John Wiley & Sons (New York), published by Wiley-Interscience and Greene Publishing Associates), based upon the present description, how to produce a cDNA clone, e.g., from total RNA via RT-PCR, how to transfect such a clone, e.g., into either primary cells or cell lines, how to select for stably transfected cells or cell lines, and to determine whether a given transfected cell or cell line overexpresses the gene of interest].

Then the overexpressing PC12 cell line can be exposed to NGF, in the presence or absence of a test agent, for several days, depending, in part, on the stability of the test agent, and the degree of neurite outgrowth can be quantitated and compared, where a test agent is an inhibitor of PTPbr7 when the degree of neurite growth is more in the test agent's presence than in its absence. Preferably, the degree of neurite growth is substantially diminished (about 50% of the growth seen in the presence of the test agent) in the absence of the test agent, where the test agent is deemed to be an inhibitor.

In another aspect of the cell-based screening assays of the present invention, PTPbr7 and MAP kinase are co-transfected into HEK 293 cells using conventional methods, overexpressing cell lines are identified, e.g., by Northern Blot analysis, and these cell lines are then treated with a growth factor capable of soliciting a detectable response in such HEK 293 cells, for a suitable period of time, e.g., several days, in the presence or absence of the test agent, and the MAP kinase phosphorylation is monitored and compared as a readout of the inhibition of PTPbr7.

Hence, the subject assays provide a simple, specific, quantitative, and relatively quick way to screen many potential inhibitors of the catalytic activity of PTPbr7. As discussed above, signaling transduction pathways generally involve several phosphorylation and dephosphorylation events; hence, as would be appreciated by those skilled in the art, the ability to administer a compound which is a specific inhibitor of the target phosphatase lessens the risk of negatively interfering with other phosphatases in the target or other pathways which may act positively toward the desired signaling.

The present assays can also be performed relatively cheaply since suitable readouts include colorimetric determinations, as opposed to the necessity of using an antibody, detecting that antibody, purifying the products to be measured, e.g., by sodium dodecyl sulfate (SDS)-polyacrylamide gel electrophoresis (PAGE), and determining the specificity of the products to be examined, e.g., by enzyme-linked immunospecific assays (ELISAs), and the like. Yet another advantage of the subject assays is the avoidance of the use of radioactive substances, where so desired. The assays of the present invention a way to minimize costs and to maximize efficiency since they can also be scaled-up to accommodate high-throughput analysis, i.e., identification of test agents from screening a compound library, and multiple test agents can be present in any given, e.g., well, as those skilled in the art will understand, based upon the present description, how to use conventional methods to deconvolute such results, to assess whether a given test agent functioned as an inhibitor of the catalytic activity of PTPbr7.

Other aspects of the present invention include pharmaceutical compositions comprising an inhibitor of the catalytic activity of PTPbr7 (or a pharmaceutically-acceptable salt thereof depending upon the nature of the test agent) and a pharmaceutically-acceptable carrier, vehicle, or diluent. Those skilled in the art based upon the present description and the nature of any given inhibitor identified by the assays of the present invention will understand how to determine a therapeutically effective dose thereof.

The pharmaceutical compositions may be manufactured using any suitable means, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in a conventional manner using one or more physiologically or pharmaceutically acceptable carriers (vehicles, or diluents) comprising excipients and auxiliaries which facilitate processing of the active compounds into preparations which can be used pharmaceutically. Proper formulation is dependent upon the route of administration chosen.

Any suitable method of administering a pharmaceutical composition to a patient may be used in the methods of treatment of the present invention, including injection, transmucosal, oral, inhalation, ocular, rectal, long acting implantation, liposomes, emulsion, or sustained release means.

For injection, the agents of the invention may be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art. For ocular administration, suspensions in an appropriate saline solution are used as is well known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained as a solid excipient, optionally grinding a resulting mixture, and processing the mixture of granules, after adding suitable auxiliaries, if desired, to obtain tablets or dragee cores. Suitable excipients include fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl cellulose, sodium carboxymethylcellulose, and/or polyvinyl-pyrrolidone (PVP). If desired, disintegrating agents may be added, such as cross-linked polyvinylpyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with fillers such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, e.g., gelatin, for use in an inhaler or insufflator, may be formulated containing a powder mix of the compound and a suitable powder base such as lactose or starch.

The compounds may be formulated for parenteral administration by injection, *e.g.*, by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active ingredient may be in powder form for constitution with a suitable vehicle, such as sterile pyrogen-free water, before use.

The compounds may also be formulated in rectal compositions such as suppositories or retention enemas, *e.g*., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

One type of pharmaceutical carrier for hydrophobic compounds of the invention is a cosolvent system comprising benzyl alcohol, a nonpolar surfactant, a water-miscible organic polymer, and an aqueous phase.

The cosolvent system may be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:5W) consists of VPD diluted 1:1 with a 5% dextrose in water solution. This co-solvent system dissolves hydrophobic compounds well, and itself produces low toxicity upon systemic administration. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of polysorbate 80; the fraction size of polyethylene glycol may be varied; other biocompatible polymers may replace polyethylene glycol, *e.g*., polyvinyl pyrrolidone; and other sugars or polysaccharides may be substituted for dextrose.

Alternatively, other delivery systems for hydrophobic pharmaceutical compounds may be employed. Liposomes and emulsions are well known examples of delivery vehicles or carriers for hydrophobic drugs. Certain organic solvents such as dimethylsulfoxide also may be employed.

Additionally, the compounds may be delivered using any suitable sustained-release system, such as semipermeable matrices of solid hydrophobic polymers containing the therapeutic agent. Various sustained-release materials have been established and are well known by those skilled in the art. Sustained-release capsules may, depending on their chemical nature, release the compounds for a prolonged period of time. Depending on the chemical nature and the biological stability of the therapeutic reagent, additional strategies for protein stabilization may be employed.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Many of the agents of the invention may be provided as salts with pharmaceutically acceptable counterions. Salts tend to be more soluble in aqueous or other protonic solvents than are the corresponding free base forms.

Other aspects of the present invention include methods of treating a condition or a disease in a mammal comprising administering to said mammal a pharmaceutical composition of the present invention.

The present invention is illustrated by the following EXAMPLES which are provided solely for the purposes of illustration and do not limit the invention. Moreover, it will also be understood that other changes and modifications that may be practiced are also part of this invention and, as such, are also within the scope of the appendant claims: those skilled in the art will recognize, or be able to ascertain without undue experimentation, equivalents to the specific embodiments of this invention described herein, and such equivalents are intended to be encompassed by the appendant claims.

### EXAMPLE 1

### PTPbr7 is a negative regulator of NGF signal transduction

Total mouse brain RNA (Strategene) was subjected to RT-PCR cloning using CTTAGCCTAGAGTGGGGTTGGCGGC as the 5' primer, and GACACTCTGGTAAATCTTCGG as the 3' primer (*CPMB*, see, in particular, Chapter 15, The Polymerase Chain Reaction, Supplement 30). The cDNA clone (full length mouse PTPbr7) was introduced into PC12 cells by transfection using Lipofectamine™ Reagent (Life Technologies) and substantially following the instructional sheet (for adherent cells, see also, *CPMB*, in particular, Chapter 9, Introduction of DNA into Mammalian Cells, Supplement 39) which specifies the concentration of DNA and reagent, cell density, transfection times, and transfection protocol. Stable cell lines were selected in (200 µg/mL) G418 (Life Technologies), as also provided by the instructional sheet, and the overexpressing cell lines were identified by Northern Blot analysis (see *CPMB*, in particular, Chapter 4, Preparation and Analysis of RNA, Chapter 4.9.1, Analysis of RNA by Northern Blot Hybridization, Supplement 26). The transfected cell lines were then treated with 50 ng/mL NGF (Life Technologies) for 6 days. The degree of neurite outgrowth was quantitated using photomicrographs where the total number of cells in the field was counted, then the cells were recounted that had one or more neurites that were greater than or equal to one cell body diameter in length, and a "% neurites" was determined.

**TABLE 1**

| **Cell Line** | **Level of PTPbr7 Expression** | **% Neurite on Day 6 (+NGF)** |
|---|---|---|
| br7-9 | none | 52 |
| br7-11 | high | 26 |
| br7-12 | high | 24 |
| br7-13 | high | 32 |

The results provided in TABLE 1 show that cell lines overexpressing PTPbr7 had about 50% fewer neurites than transfected cells which did not overexpress the transgene (br7-9), and untransfected PC12 cells (data not shown), thereby evidencing an activity for PTPbr7 in the negative regulation of NGF signal transduction. Based upon the present description, those skilled in the art will understand how to the above cell lines in the assays of the present invention to ascertain the effects of including a test agent or putative inhibitor, or any other effector of PTPbr-7 of interest as the case may be.

### EXAMPLE 2

### PTPbr7 inhibition assay: peptide substrate

The ability of a test agent (positive control, non-specific tyrosine phosphatase inhibitor, sodium orthovanadate) to inhibit the dephosphorylation of a peptide having a phosphotyrosine residue by a PTPbr7 fusion protein was determined as follows.GST-PTPbr7 (4 µg, PanVera) is mixed together with the peptide [ENDpYINASL] (0.1 mM, American Peptide Company), with and without sodium orthovanadate (1 mM, SIGMA®-Aldrich), in a total volume of 25 µL assay buffer (50 mM Tris, 0.15 M NaCI, 5 mM DTT, 0.1% BSA), pH 7.4, at 25°C, in separate wells of a ½ volume 96-well microtiter plate (COSTAR #3690 plate), for 30 min.

The reactions were then terminated by the addition of 100 µL of MG/AM stop solution. MG (Malachite Green) solution was obtained from SIGMA®-Aldrich cat no. M9636, and can be prepared by dissolving 500 mg/L in dH₂O, filtering the solution with a Whatman #1 filter, and storing at 4°C. AM (Ammonium Molybdate) solution was obtained from SIGMA®-Aldrich cat. no. A7302, 42 g/L in 4 M HCI (345 mL concentration HCI/L, stored at 4°C). MG/AM mixture was prepared by mixing one part AM to 3 parts MG, stirring for at least 30 min, at room temperature, filtering with a Whatman #5, and storing at 4°C (for up to 6 months). MG/AM stop solution was prepared by adding 0.01% Tween-20 to an aliquot of MG/AM mixture (warmed to room temperature).

The plate is then incubated at room temperature for 15 min and, after removing any bubbles, the optical density of the solution in each well was read at A₆₂₀ (620 nm, spectrophotometer). A standard curve from 100 pmols to 5000 pmols in 25 µL total (in MG/AM stop solution) is used to calculate the amount of PQ produced by the enzyme reaction (in each well). The optical density at 620 nm in the presence and absence of the test agent (in this example, sodium orthovanadate) was then compared. In the absence of the sodium orthovanadate, the optical density 620 nm was 0.65 and, in its presence, 0.10.

Hence, the catalytic activity of the PTPbr7 is inhibitable and, as such, the assays of the present invention, including the embodiment described in this EXAMPLE, can be used to identify a test agent as an inhibitor.

### EXAMPLE 3

### PTPbr7 inhibition assay: fluorogenic substrate

The ability of a test agent (positive control, non-specific tyrosine phosphatase inhibitor, sodium orthovanadate) to inhibit the dephosphorylation of a fluorogenic substrate by a PTPbr7 fusion protein was determined as follows.

The substrate (O-methyl fluorophosphate) was dissolved at 1 mM in 100% DMSO and diluted to 12.5µM in assay buffer (50 mM HEPES (pH 7.2), 100 mM KCI, 2.5 mM EGTA, 2.5 mM MgCl₂, 1 mg/mL BSA and 5 mM DTT). 80 µL of the diluted substrate (final concentration of 10 µM) was then added to each of two wells of a 96-well microtiter plate (COSTAR #3690 plate), followed by, in one of the wells, sodium orthovanadate (1 mM, final concentration), and in both wells, 20 µL of GST-PTPbr7 (4 µg, PanVera) to give a final enzyme concentration in each of the assays of 4 µg/mL. The reactions were incubated at room temperature for 30 min. For each reaction, the fluorescent emission was measured by excitation at 485 nm and emission at 535 nm using a Victor II plate reader (Packard). The presence of the test agent (sodium orthovanadate) in the reaction caused a decrease in the fluorescent signal (1250.00) compared to the reaction in the absence of inhibitor (4100.00). Hence, the catalytic activity of the PTPbr7 is inhibitable and, as such, the assays of the present invention, including the embodiment described in this EXAMPLE, can be used to identify a test agent as an inhibitor.

Those skilled in the art will further understand from these EXAMPLES that the assays described in the present description and attendant claims provide powerful tools for identifying inhibitors of the catalytic activity of PTPbr7, thus enabling the art to treat diseases and conditions, e.g., neurodegenerative diseases and conditions, that would benefit from, for example, the inhibition of PTPbr7, a restoration of NGF signaling, and/or improved survival, differentiation and maintenance of specific populations of neurons, e.g., cholinergic neurons, e.g., in the basal forebrain, shown to be particularly at risk for degeneration in AD.

As mentioned above, variations and modifications commensurate with the above teachings, and the skill or knowledge in the relevant art are within the scope of the present invention.

## Claims

1. A method for identifying an agent that inhibits the catalytc activity of PTPbr7, comprising the steps of:
(a) combining, in a first cocktail, an amount of a PTPbr7, an amount of a substrate capable of being dephosphorylated by PTPbr7, and an amount of a test agent, in an assay buffer comprising a reducing agent;
(b) preparing a second cocktail comprising all of the ingredients of said first cocktail except for said test agent;
(c) incubating said first and second cocktails under conditions suitable to allow for a substantial amount of dephosphorylation of said substrate by said PTPbr7;
(d) terminating said incubation;
(e) quantitating the amount of dephosphorylation in each of said cocktails; and
(f) comparing said amounts of dephosphorylation;
where a PTPbr7 inhibitor is a test agent whose presence results in less dephosphorylation than its absence.

2. The method as defined in claim **1** wherein said PTPbr7 is a fusion protein comprising the catalytic domain of PTPbr7 and a heterologous sequence.

3. The method as defined in claim **2** wherein said heterologous sequence is glutathione-S-transferase or a six-histidine residue tag.

4. The method as defined in claim **1** wherein said substrate is a small molecule mimetic.

5. The method as defined in claim **1** wherein said substrate is [ENDpYINASL] or [DHTGFLTEpYVATRW].

6. The method as defined in claim **1** wherein said substrate is MAPK-P.

7. The method as defined in claim **1** wherein said amount of said assay buffer is about 25 µL, said assay buffer comprises about 50 mM Tris, about 0.15 M NaCI, about 5 mM DTT, and about 0.1% BSA, at about pH 7.4, said terminating is by adding a stop solution comprising malachite green dye, ammonium molybdate, and Tween-20 to said cocktails, and waiting about 15 min, and said quantitating is by measuring the optical density at 620 nm of each of said stopped cocktails using a spectrophotometer, and comparing the optical density values with a set of standards comprising varied amounts of inorganic phosphate in said stop solution.

8. A pharmaceutical composition comprising an inhibitor of the catalytic activity of PTPbr7 as defined in claim **1** and a pharmaceutically acceptable carrier, vehicle, or diluent.

9. A method for identifying an agent that inhibits the catalytic activity of PTPbr7, comprising the steps of:
(a) combining, in a first cocktail, an amount of a PTPbr7, an amount of a substrate capable of being dephosphorylated by PTPbr7, and an amount of a test agent, in an assay buffer comprising a reducing agent;
(b) preparing a second cocktail comprising all of the ingredients ofsaid first cocktail except for said test agent;
(c) incubating said first and second cocktails under conditions suitable to allow for a substantial amount of dephosphorylation of said substrate by said PTPbr7;
(d) quantitating the amount of dephosphorylation in each of said cocktails; and
(e) comparing said amounts of dephosphorylation;
where a PTPbr7 inhibitor is a test agent whose presence results in less dephosphorylation than its absence.

10. A pharmaceutical composition comprising an inhibitor of the catalytic activity of PTPbr7 as defined in claim 9 and a pharmaceutically acceptable carrier, vehicle, or diluent.

11. A method of screening for an agent that inhibits the catalytic activity of PTPbr7, comprising the steps of: exposing cells or a cell line expressing PTPbr7 and capable of responding to NGF, to an amount of NGF, in the presence and absence of a test agent, under conditions suitable for a detectable NGF response to occur in the presence of a PTPbr7 inhibitor, detecting said response, and comparing said response, where a PTPbr7 inhibitor is a test agent whose presence results in more of an NGF response than its absence.

12. A pharmaceutical composition comprising an inhibitor of the catalytic activity of PTPbr7 as defined in claim **11** and a pharmaceutically acceptable carrier, vehicle, or diluent.

13. A method of screening for an agent that inhibits the catalytic activity of PTPbr7, comprising the steps of: exposing a cell line expressing PTPbr7 and capable of responding to NGF, to an amount of NGF, in the presence and absence of a test agent, under conditions suitable for detectable neurite growth to occur in the presence of a PTPbr7 inhibitor, detecting said neurite growth, and comparing said neurite growth, where a PTPbr7 inhibitor is a test agent whose presence results in more neurite growth than its absence.

14. The method as defined in claim **13** wherein said cell line is a PC12 cell line, or a PC12 cell line transfected with a PTPbr7.

15. The method as defined in claim **14** wherein said transfected PC12 cell line overexpresses said PTPbr7.
